# EUROPEAN PATENT APPLICATION

(11) **EP 3 037 029 A1**
(43) Date of publication of application: **29.06.2016**
(21) Application number: 14837393.9
(22) Date of filing: 07.08.2014
(51) Int. Cl.: A61B 1/00

(54) **LOCATION DETECTION DEVICE AND LOCATION DETECTION SYSTEM**

(30) Priority: 22.08.2013 JP 2013172545
(71) Applicant: OLYMPUS CORPORATION, Shibuya-ku Tokyo 151-0072 (JP)
(72) Inventor: CHIBA, Atsushi, Tokyo 151-0072 (JP)
(74) Representative: Gunzelmann, Rainer
(86) International application number: PCT/JP2014/070949
(87) International publication number: WO 2015/025731

(57) **Abstract**

Provided is a position detection device and a position detection system capable of accurately detecting a three-dimensional position of a capsule medical device and achieving space saving in the entire system. The position detection device detects a position of a capsule endoscope 10 inside which a coil for generating a magnetic field is disposed. The position detection device includes a plurality of sensing coils 32 each configured to detect the magnetic field generated by the coil and to output a detection signal, and a position calculation unit 562b configured to calculate the position of the capsule endoscope 10 based on the detection signal output form each sensing coil 32. The sensing coils 32 are arranged in an arrangement region that is a closed region on a panel 31. The arrangement region includes a detection target region formed by projecting a closed space for detecting the capsule endoscope 10 on the panel 31, and is larger than the detection target region by a predetermined ratio.

## Description

### Field

The present invention relates to a position detection device and a position detection system for detecting a position of a capsule medical device introduced into a subject.

### Background

Conventionally, a capsule medical device that is introduced into a subject to acquire various pieces of information about the inside of the subject or to deliver a medical agent into the subject has been developed. As an example, in the field of endoscope, there is known a capsule endoscope having a size enough to be introduced into the digestive tract (lumen) of a subject. The capsule endoscope has an imaging function and a wireless communication function inside a capsule-shaped casing. The capsule endoscope is swallowed through the mouth of a subject, then captures the inside of the digestive tract while moving inside the digestive tract by, for example, peristaltic movement, and sequentially wirelessly transmits image data of images inside the organs of the subject (hereinafter also referred to as in-vivo images). The wirelessly-transmitted image data is received by a receiving device which is disposed outside the subject.
The received image data is then captured by an image processing device such as a work station and subjected to predetermined image processing. Accordingly, an in-vivo image of the subject can be played/displayed as a still image or a moving image in the image processing device.

Further, a system for detecting the position of a capsule medical device inside a subject has been developed to specify a part of the subject that appears on an in-vivo image. For example, Patent Literature 1 discloses a technique in which a coil (marker coil) for generating a magnetic field is disposed inside a capsule medical device, the magnetic field generated by the marker coil is detected by a coil for magnetic field detection (hereinafter referred to as a sensing coil) disposed outside a subject, and a position of the capsule medical device is estimated based on strength of the detected magnetic field.

### Citation List

### Patent Literature

Patent Literature 1: Japanese Patent Application Laid-open No. 2008-132047

### Summary

### Technical Problem

In order to improve the accuracy of detecting a capsule medical device inside a subject, the strength of a magnetic field generated from the capsule medical device may be increased or the number of sensing coils may be increased. However, in the former case, power consumption in the capsule medical device increases, which shortens the life of a battery incorporated therein. Thus, the former case is not preferred. On the other hand, in the latter case, the scale of a processing circuit which processes output signals from the sensing coils increases, which disadvantageously causes an increase in the size of the system and an increase in the power consumption.

In Patent Literature 1, a plurality of panels, on each of which a plurality of sensing coils is arranged, is three-dimensionally assembled and arranged around a subject (refer to FIG. 9 of Patent Literature 1). Also in this case, a three-dimensionally large space is required around a subject, which results in an increase in the size of the entire system.

The present invention has been made in view of the foregoing, and an object of the invention is to provide a position detection device and a position detection system capable of accurately detecting the position of a capsule medical device introduced into a subject and achieving space saving in the entire system.

### Solution to Problem

In order to solve the above described problems and achieve the object, a position detection device according to the invention detects a position of a capsule medical device having therein a magnetic field generator for generating a magnetic field. The position detection device includes: a plurality of magnetic field detection units each configured to detect the magnetic field generated by the magnetic field generator and to output a detection signal; and a position calculation unit configured to calculate the position of the capsule medical device based on the detection signal output from each of the magnetic field detection units. The magnetic field detection units are arranged on a single plane, the plane having: a first region formed by projecting a closed detection target space for detecting the capsule medical device on the plane; and a second region that is a closed region on the plane and includes the first region and is larger than the first region by a predetermined ratio. The magnetic field detection units are arranged in the second region.

In the above-described position detection device, wherein at least one of the magnetic field detection units is arranged in a third region located between an outer periphery of the first region and an outer periphery of the second region.

In the above-described position detection device, at least one of the magnetic field detection units is arranged in the first region and at least another one of the magnetic field detection units is arranged in the third region.

In the above-described position detection device, at least some of the magnetic field detection units are arranged with directions for detecting the magnetic field parallel to an axis perpendicular to the plane.

In the above-described position detection device, at least some of the magnetic field detection units are arranged in such a manner that three of the magnetic field detection units whose directions for detecting the magnetic field are respectively parallel to two axes perpendicular to each other within the plane and an axis perpendicular to the plane are defined as one set.

In the above-described position detection device, some of the magnetic field detection units are arranged in the first region with the directions for detecting the magnetic field parallel to the axis perpendicular to the plane. Some other of the magnetic field detection units are arranged in a third region located between an outer periphery of the first region and an outer periphery of the second region in such a manner that three of the magnetic field detection units whose directions for detecting the magnetic field are respectively parallel to the two axes perpendicular to each other within the plane and the axis perpendicular to the plane are defined as one set.

In the above-described position detection device, nearest neighbor distances between the magnetic field detection units are constant.

In the above-described position detection device, a nearest neighbor distance between any two of the magnetic field detection units in a peripheral part of the second region is shorter than a nearest neighbor distance between any two of the magnetic field detection units in a central part of the second region.

In the above-described position detection device, the second region has a quadrangular shape, and a nearest neighbor distance between any two of the magnetic field detection units at a corner of the quadrangular shape of the second region is shorter than a nearest neighbor distance between any two of the magnetic field detection units in a central part of the second region.

In the above-described position detection device, the ratio is 120% or more.

In the above-described position detection device, the ratio is 120% or more and 200% or less.

In the above-described position detection device, the ratio is 120% or more and 140% or less.

A position detection system according to the invention includes the position detection device and a capsule medical device having therein a magnetic field generator configured to generate a magnetic field.

The above-described position detection system further includes a bed for placing thereon a subject into which the capsule medical device is configured to be introduced. The magnetic field detection units are arranged under the bed integrally with the bed with the plane parallel to a surface of the bed on which the subject is configured to be placed.

In the above-described position detection system, the capsule medical device further has therein a permanent magnet. The position detection system further includes: a guidance magnetic field generator that is disposed outside a subject into which the capsule medical device is configured to be introduced, the guidance magnetic field generator being configured to generate a magnetic field in a space including the first region; and a guidance magnetic field controller configured to at least one of translate and rotate the guidance magnetic field generator to change a guidance magnetic field at a position of the capsule medical device, and thereby to guide the capsule medical device in the subject.

In the above-described position detection system, the first region is smaller than a range in which the guidance magnetic field generator is movable within a horizontal plane.

In the above-described position detection system, the magnetic field generator is configured to generate an alternating magnetic field having a predetermined frequency component.

### Advantageous Effects of Invention

According to the present invention, a plurality of magnetic field detection units is arranged on a plane, the plane having: a first region formed by projecting a detection space for detecting the capsule medical device on the plane; and a second region that is a closed region on the plane and includes the first region and is larger than the first region by a predetermined ratio, and the magnetic field detection units are arranged in the second region. Thus, it is possible to accurately detect the position of the capsule medical device and to achieve space saving in the entire system.

### Brief Description of Drawings

FIG. 1 is a schematic diagram illustrating an example of the configuration of a position detection system according to a first embodiment of the present invention.
FIG. 2 is a schematic diagram illustrating an example of the internal structure of a capsule endoscope illustrated in FIG. 1.
FIG. 3 is a schematic diagram illustrating an example of the configuration of a guidance magnetic field generation device illustrated in FIG. 1.
FIG. 4 is a schematic diagram illustrating an example of the arrangement of sensing coils in a position detection device according to the first embodiment (with nine sensing coils and a detection target region of 100%).
FIG. 5 is a schematic diagram illustrating an example of the arrangement of sensing coils in the position detection device according to the first embodiment (with eight sensing coils and a detection target region of 140%).
FIG. 6 is a schematic diagram illustrating an example of the arrangement of sensing coils in the position detection device according to the first embodiment (with 13 sensing coils).
FIG. 7 is a schematic diagram illustrating an example of the arrangement of sensing coils in the position detection device according to the first embodiment (with 21 sensing coils).
FIG. 8 is a schematic diagram illustrating an example of the arrangement of sensing coils in the position detection device according to the first embodiment (with 16 sensing coils).
FIG. 9 is a schematic diagram illustrating an example of the arrangement of sensing coils in the position detection device according to the first embodiment (with 25 sensing coils).
FIG. 10 is a graph illustrating a result of a simulation of a variation in detection position of the capsule endoscope when the ratio of an arrangement region to the detection target region is changed in the arrangement of sensing coils illustrated in each of FIGS. 4 to 9.
FIG. 11 is a schematic diagram illustrating the arrangement of sensing coils in a first modification of the first embodiment.
FIG. 12 is a schematic diagram illustrating an example of the arrangement of sensing coils in a position detection device according to a second embodiment of the present invention (with 12 sensing coils).
FIG. 13 is a schematic diagram illustrating an example of the arrangement of sensing coils in the position detection device according to the second embodiment of the present invention (with 15 sensing coils).
FIG. 14 is a schematic diagram illustrating an example of the arrangement of sensing coils in the position detection device according to the second embodiment of the present invention (with 18 sensing coils).
FIG. 15 is a schematic diagram illustrating an example of the arrangement of sensing coils in the position detection device according to the second embodiment of the present invention (with 21 sensing coils).
FIG. 16 is a schematic diagram illustrating an example of the arrangement of sensing coils in the position detection device according to the second embodiment of the present invention (with 24 sensing coils).
FIG. 17 is a perspective view of a coil set illustrated in FIGS. 12 to 16.
FIG. 18 is a graph illustrating a result of a simulation of a variation in detection position of the capsule endoscope when the ratio of the arrangement region to the detection target region is changed in the arrangement of sensing coils illustrated in each of FIGS. 12 to 16.
FIG. 19 is a schematic diagram illustrating an example of the arrangement of sensing coils in a position detection device according to a third embodiment.
FIG. 20 is a schematic diagram illustrating a third modification of the position detection systems according to the first to third embodiments of the present invention.
FIG. 21 is a schematic diagram illustrating a fourth modification of the position detection systems according to the first to third embodiments of the present invention. Description of Embodiments

Hereinbelow, a position detection device and a position detection system according to some embodiments of the present invention will be described with reference to the drawings. Although, in the following description, a capsule endoscope that is introduced into a subject through the mouth and captures the inside of the subject (the inside of the lumen) is described as an embodiment of a capsule medical device to be detected by the position detection device and the position detection system according to the embodiments of the present invention, the present invention is not limited by the embodiment. That is, the present invention is applicable to position detection for various capsule-shaped medical devices, such as a capsule endoscope that moves inside the lumen of a subject from the esophagus through the anus, a capsule medical device that delivers, for example, a medical agent into a subject, and a capsule medical device provided with a PH sensor which measures PH inside a subject.

In the following description, each of the drawings merely illustrates a shape, size, and positional relationship schematically to the degree of allowing understanding of the contents of the present invention. Thus, the present invention is not limited only to the shape, size, and positional relationship illustrated in each of the drawings. The same reference signs are used to designate the same elements throughout the drawings.

### (FIRST EMBODIMENT)

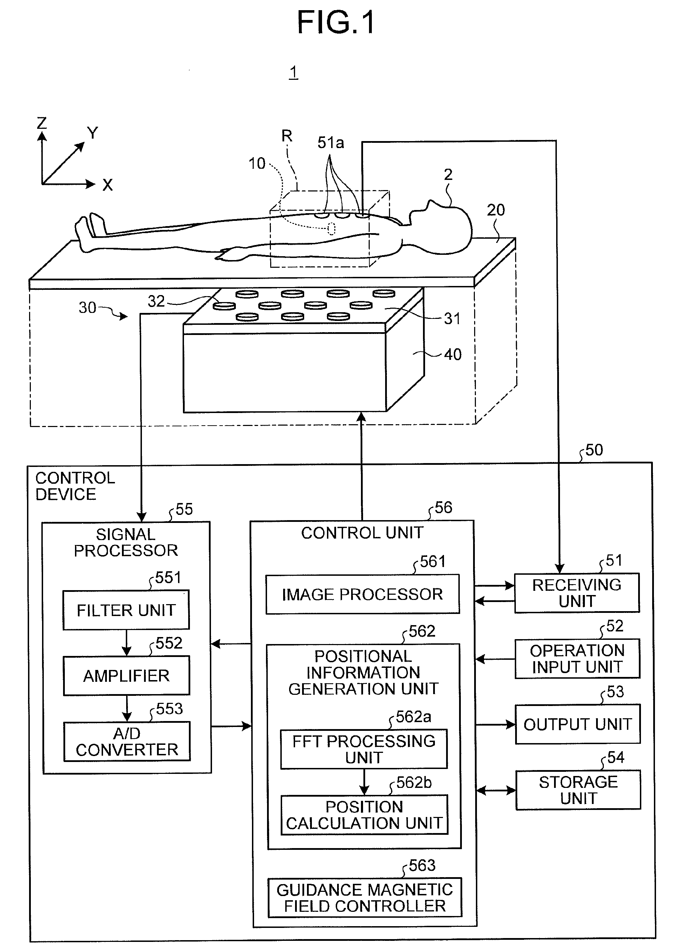

FIG. 1 is a diagram illustrating an example of the configuration of a position detection system according to a first embodiment of the present invention. As illustrated in FIG. 1, the position detection system 1 according to the first embodiment includes a capsule endoscope 10 which transmits image data acquired by imaging the inside of a subject 2 so as to be superimposed on a wireless signal as an example of a capsule medical device to be introduced into the lumen of the subject 2, a magnetic field detection device 30 which is placed under a bed 20 on which the subject 2 is placed and detects an alternating magnetic field generated by the capsule endoscope 10, a guidance magnetic field generation device 40 which generates a guidance magnetic field for guiding the capsule endoscope 10, and a control device 50 which detects the position of the capsule endoscope 10 based on an alternating magnetic field detected by the magnetic field detection device 30 and controls the guidance magnetic field generation device 40 to guide the capsule endoscope 10 inside the subject 2.

The bed 20 is placed with an upper face (a surface on which the subject 2 is placed) parallel to a horizontal plane (a plane perpendicular to the gravity direction). In the following description, a longitudinal direction of the bed 20 is defined as an X direction, a transverse direction of the bed 20 is defined as a Y direction, and a vertical direction (gravity direction) is defined as a Z direction. The magnetic field detection device 30 and the control device 50 together constitute a position detection device capable of detecting the capsule endoscope 10 in a detection target space R on the bed 20. A three-dimensional closed region that includes a range in which the capsule endoscope 10 is movable inside the subject 2 (that is, a range of observation target organs) is previously set as the detection target space R.

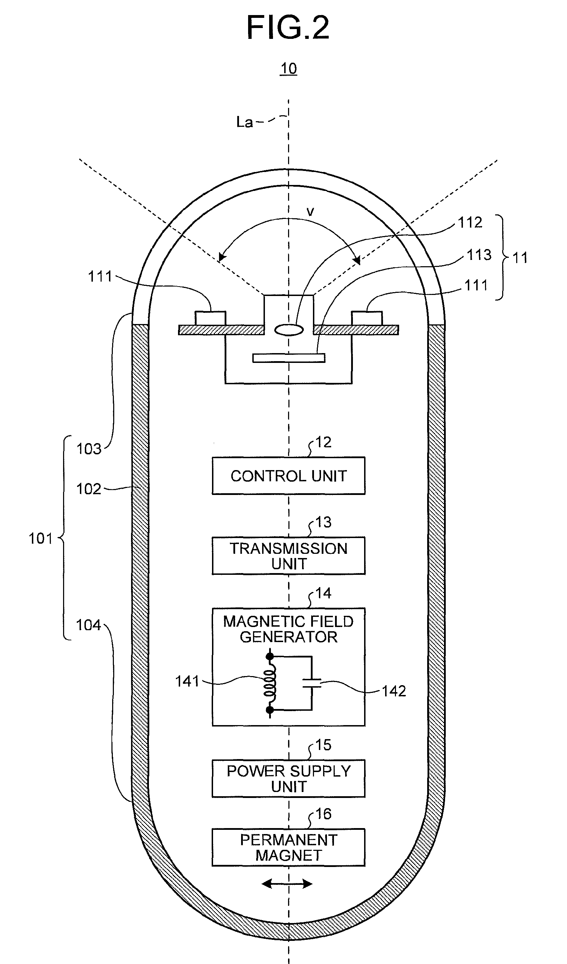

FIG. 2 is a schematic diagram illustrating an example of the internal structure of the capsule endoscope 10 illustrated in FIG. 1. As illustrated in FIG. 2, the capsule endoscope 10 includes a capsule-shaped casing 101 having a size enough to be easily introduced into the lumen of the subject 2, an imaging unit 11 which is housed inside the casing 101 and captures the inside of the subject 2 to acquire an imaging signal, a control unit 12 which controls the operation of each unit of the capsule endoscope 10 including the imaging unit 11 and performs predetermined signal processing on an imaging signal acquired by the imaging unit 11, a transmission unit 13 which wirelessly transmits the imaging signal on which the signal processing has been performed, a magnetic field generator 14 which generates an alternating magnetic field for detecting the position of the capsule endoscope 10, a power supply unit 15 which supplies electric power to each unit of the capsule endoscope 10, and a permanent magnet 16.

The casing 101 is an outer casing having a size enough to be introduced into the organs of the subject 2, and is formed by blocking both opening ends of a cylindrical casing 102 having a cylindrical shape with dome-shaped casings 103 and 104. The cylindrical casing 102 is formed of a colored member that is substantially opaque to visible light. At least either of the dome-shaped casing 103 or the dome-shaped casing 104 (in FIG. 2, the dome-shaped casing 103 located at a side corresponding to the imaging unit 11) is formed of an optical member that is transparent to light having a predetermined wavelength band such as visible light. Although, in FIG. 2, only one imaging unit 11 is disposed at the side corresponding to the dome-shaped casing 103, two imaging units 11 may be provided. In this case, the dome-shaped casing 104 is also formed of a transparent optical member. The casing 101 having such a configuration liquid-tightly encloses therein the imaging unit 11, the control unit 12, the transmission unit 13, the magnetic field generator 14, the power supply unit 15, and the permanent magnet 16.

The imaging unit 11 is an information acquiring unit which acquires an imaging signal as information about the subject 2. The imaging unit 11 includes an illumination unit 111 which includes a light emitting element such as an LED and a drive circuit (not illustrated) which drives the light emitting element, an optical system 112 such as a condenser lens, and an imager 113 which includes an imaging element such as a CMOS image sensor and a CCD and a drive circuit (not illustrated) which drives the imaging element. The illumination unit 111 emits illumination light such as white light to an imaging viewing field of the imager 113 to illuminate the subject 2 within the imaging viewing field v through the dome-shaped casing 103. The optical system 112 is disposed with an optical axis La coincident with a long axis of the casing 101. The optical system 112 concentrates reflected light from the subject 2 within the imaging viewing field v to form an image on an imaging surface of the imager 113. The imager 113 photoelectric-converts an optical signal that indicates an image of the subject 2 formed on the imaging surface to generate an imaging signal.

If two imaging units 11 are employed, the imaging units 11 are disposed corresponding to the respective dome-shaped casings 103 and 104 on both ends of the casing 101 with both optical axes La of two optical systems 112 coincident with the long axis of the casing 101.

The control unit 12 controls the imager 113 to operate at a predetermined period (imaging frame rate) and controls the illumination unit 111 to emit light in synchronization with the imaging frame rate. The control unit 12 performs A/D conversion or another predetermined signal processing on an imaging signal generated by the imaging unit 11 to generate image data. The control unit 12 controls the power supply unit 15 to supply electric power to the magnetic field generator 14 to allow the magnetic field generator 14 to generate an alternating magnetic field.

The transmission unit 13 includes a transmission antenna (not illustrated). The transmission unit 13 acquires image data on which signal processing has been performed by the control unit 12 and related information and performs modulation processing, and sequentially wirelessly transmits the image data and the related information to the outside through the transmission antenna.

The magnetic field generator 14 is included in a resonance circuit. The magnetic field generator 14 includes a transmission coil 141 which generates a magnetic field by current flowing thereto and a capacitor 142 which forms the resonance circuit together with the transmission coil 141. The magnetic field generator 14 generates an alternating magnetic field having a predetermined frequency upon receiving power supply from the power supply unit 15.

The power supply unit 15 is implemented by, for example, a button battery and a switch unit such as a magnetic switch. The power supply unit 15 switches between on and off states of its own by a magnetic field applied from the outside. During an on state, the power supply unit 15 supplies power to each unit of the capsule endoscope 10. During an off state, the power supply unit 15 stops power supply to each unit of the capsule endoscope 10.

The permanent magnet 16 enables magnetic guidance of the capsule endoscope 10 by a magnetic field generated by the guidance magnetic field generation device 40. The permanent magnet 16 is disposed in a fixed manner inside the capsule-shaped casing 101 with a magnetization direction inclined with respect to the long axis La. In FIG. 2, the magnetization direction of the permanent magnet 16 is indicated by an arrow. In the first embodiment, the permanent magnet 16 is disposed with the magnetization direction perpendicular to the long axis La. The permanent magnet 16 operates following a magnetic field applied from the outside. As a result, magnetic guidance of the capsule endoscope 10 by the guidance magnetic field generation device 40 is achieved.

Referring again to FIG. 1, the magnetic field detection device 30 includes a planar panel 31 and a plurality of sensing coils 32 which is arranged on a principal face of the panel 31. Each of the sensing coils 32 receives an alternating magnetic field generated from the capsule endoscope 10 and outputs a detection signal. Each of the sensing coils 32 is a magnetic field detection unit including, for example, a coil-spring-shaped tubular coil having an opening diameter of approximately 30 to 40 mm and a height of approximately 5 mm.

The magnetic field detection device 30 is placed near the subject 2 during an examination. In the first embodiment, the magnetic field detection device 30 is placed under the bed 20 with the principal face of the panel 31 horizontal (parallel to the surface on which the subject 2 is placed). The arrangement of the sensing coils 32 on the panel 31 will be described below. In the first embodiment, the magnetic field detection device 30 and the control device 50 (described below) together constitute the position detection device.

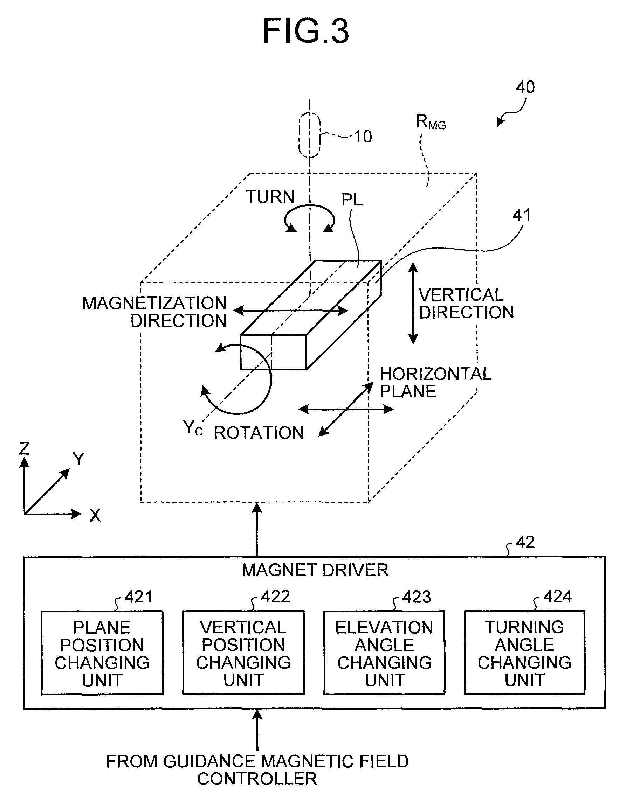

FIG. 3 is a schematic diagram illustrating an example of the configuration of the guidance magnetic field generation device 40. As illustrated in FIG. 3, the guidance magnetic field generation device 40 generates a guidance magnetic field for changing the position, the inclination angle of the long axis La with respect to the vertical direction, and the directional angle of the capsule endoscope 10 introduced into the subject 2 relative to the subject 2. More specifically, the guidance magnetic field generation device 40 incldues an external permanent magnet 41 as a guidance magnetic field generator which generates a magnetic field, and a magnet driver 42 which changes the position and the posture of the external permanent magnet 41. The magnet driver 42 includes a plane position changing unit 421, a vertical position changing unit 422, an elevation angle changing unit 423, and a turning angle changing unit 424.

The external permanent magnet 41 is preferably implemented by a bar magnet having a rectangular parallelepiped shape. The external permanent magnet 41 locks the capsule endoscope 10 within a region formed by projecting one of four planes parallel to the magnetization direction of the external permanent magnet 41 (hereinafter also referred to as a capsule facing plane PL) on a horizontal plane. Hereinbelow, a state in which the capsule facing plane PL is horizontal and the magnetization direction is parallel to the X axis is referred to as reference arrangement.

The plane position changing unit 421 translates the external permanent magnet 41 within an XY plane. That is, the external permanent magnet 41 moves within the horizontal plane with a relative position between two magnetic poles magnetized in the external permanent magnet 41 maintained.

The vertical position changing unit 422 translates the external permanent magnet 41 along the Z direction. That is, the external permanent magnet 41 moves along the vertical direction with a relative position between two magnetic poles magnetized in the external permanent magnet 41 maintained.

The elevation angle changing unit 423 rotates the external permanent magnet 41 within a vertical plane that includes the magnetization direction of the external permanent magnet 41 to change the angle of the magnetization direction with respect to the horizontal plane. In other words, the elevation angle changing unit 423 rotates the external permanent magnet 41 with respect to an axis that is parallel to the capsule facing plane PL and perpendicular to the magnetization direction, and passes through the center of the external permanent magnet 41 (hereinafter referred to as a rotation axis Y_{c}). Hereinbelow, an angle between the external permanent magnet 41 and the horizontal plane is referred to as an elevation angle.

The turning angle changing unit 424 rotates the external permanent magnet 41 with respect to a vertical direction axis that passes through the center of the external permanent magnet 41. Hereinbelow, a rotational movement of the external permanent magnet 41 with respect to the vertical direction axis is referred to as a turning movement. Further, an angle of the turn of the external permanent magnet 41 with respect to the reference arrangement is referred to as a turning angle.

The inclination angle and the directional angle of the capsule endoscope 10 locked by a magnetic field generated by the external permanent magnet 41 can be changed by turning the external permanent magnet 41 by the turning angle changing unit 424 to change the angle of the rotation axis Y_{c} with respect to the reference arrangement and, in this state, rotating the external permanent magnet 41 with respect to the rotation axis Y_{c} by the elevation angle changing unit 423.

The operation of each unit of the magnet driver 42 is controlled by a guidance magnetic field controller 563 (described below). A movable range R_{MG} of the external permanent magnet 41 within the horizontal plane is previously set so as to move the capsule endoscope 10 to a position desired by a user within the detection target space R illustrated in FIG. 1. The movable range R_{MG} is equal to or wider than a detection target region formed by projecting the detection target space R on the horizontal plane.

As illustrated in FIG. 1, the control device 50 includes a receiving unit 51 which receives a wireless signal transmitted from the capsule endoscope 10 through receiving antennas 51a, an operation input unit 52 which is used for inputting various pieces of information and instructions to the control device 50, an output unit 53 which outputs, for example, various pieces of information processed by the control device 50 to a display device or the like so as to be displayed thereon, a signal processor 55 which performs various kinds of signal processing on a detection signal output from each of the sensing coils 32 to generate magnetic field information, a storage unit 54, and a control unit 56 which controls the operation of these units.

In an examination using the capsule endoscope 10, the plurality of receiving antennas 51a which receive a wireless signal transmitted from the capsule endoscope 10 are stuck to the body surface of the subject 2. The receiving unit 51 selects, among the receiving antennas 51a, one having the highest reception strength to the wireless signal. The receiving unit 51 performs demodulation processing on a wireless signal received through the selected receiving antenna 51a to acquire image data of an in-vivo image and related information.

The operation input unit 52 is implemented by, for example, an input device such as various buttons, switches, and keyboards, a pointing device such as a mouth and a touch panel, or a joystick. The operation input unit 52 inputs various pieces of information to the control unit 56 in accordance with an input operation by a user. Examples of information input by the operation input unit 52 include information for guiding the capsule endoscope 10 to a position and a posture desired by a user (hereinafter referred to as guidance instruction information).

The output unit 53 includes various displays such as a liquid crystal display and an organic electro luminescence (EL) display. The output unit 53 displays various pieces of information input from the operation input unit 52, an in-vivo image of the subject 2, and positional information of the capsule endoscope 10 when an in-vivo image is captured on a screen.

The storage unit 54 is implemented by using a storage medium, which rewritably stores information, such as a flash memory and a hard disk and a writing and reading device. The storage unit 54 stores, for example, various programs and various parameters used by the control unit 56 to control each unit of the control device 50, image data of an in-vivo image captured by the capsule endoscope 10, and positional information of the capsule endoscope 10 inside the subject 2.

The signal processor 55 includes a filter unit 551 which shapes a waveform of a detection signal output from the magnetic field detection device 30, an amplifier 552, and an A/D converter 553 which performs A/D conversion on a detection signal to generate detection data. There are an alternating magnetic field generated from the capsule endoscope 10 and a guidance magnetic field formed by the guidance magnetic field generation device 40 in a space in which the magnetic field detection device 30 is capable of detecting a magnetic field. However, since both the magnetic fields have completely different frequencies, no interference between the magnetic fields occurs.

The control unit 56 is configured by using, for example, a central processing unit (CPU). The control unit 56 reads programs from the storage unit 54 to perform instruction or data transfer to the units constituting the control device 50 to totally control the operation of the control device 50. The control unit 56 includes an image processor 561, a positional information generation unit 562, and a guidance magnetic field controller 563.

The image processor 561 performs predetermined image processing such as white balance processing, demosaicing, gamma conversion, and smoothing (noise removal) on image data input from the receiving unit 51 to generate image data for display.

The positional information generation unit 562 acquires information (positional information) that indicates the position of the capsule endoscope 10 based on magnetic field detection data output from the signal processor 55. More specifically, the positional information generation unit 562 includes: an FFT processing unit 562a which performs fast Fourier transformation processing (hereinafter referred to as FFT processing) on detection data output from the A/D converter 553 to extract magnetic field information such as the amplitude and the phase of an alternating magnetic field; and a position calculation unit 562b which calculates the position of the capsule endoscope 10 based on the magnetic field information extracted by the FFT processing unit 562a.

The guidance magnetic field controller 563 controls the operation of each unit of the magnet driver 42 so as to bring the capsule endoscope 10 into a position and a posture desired by a user in accordance with positional information acquired by the positional information generation unit 562 and guidance instruction information input from the operation input unit 52. That is, the guidance magnetic field controller 563 changes the position, the elevation angle, and the turning angle of the external permanent magnet 41 to change magnetic gradient in a space that includes the position of the capsule endoscope 10, thereby guiding the capsule endoscope 10.

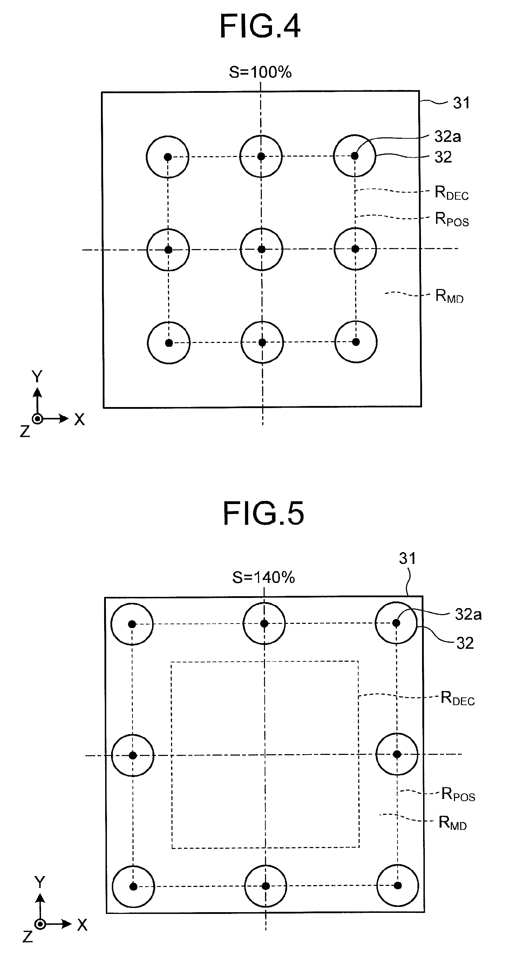

Next, the arrangement of the sensing coils 32 will be described. FIGS. 4 and 5 are schematic diagrams illustrating examples of the arrangement of a plurality of sensing coils 32 in the position detection device according to the first embodiment.

In the first embodiment, a plurality of sensing coils 32 is arranged on the panel 31 which is a single plane to achieve space saving in the position detection system. The accuracy of detecting the position of the capsule endoscope 10 is determined by a signal-to-noise ratio (SN ratio) of a magnetic field detected by each of the sensing coils 32 and arrangement conditions of the sensing coils 32. Reference will now be made below to arrangement of sensing coils 32 that enables a reduction in an arrangement space of the sensing coils 32 and position detection for the capsule endoscope 10 with less error.

The sensing coils 32 are arranged in an arrangement region (second region) R_{POS} which is a closed region on the panel 31. The arrangement region R_{POS} includes a detection target region R_{DEC} (first region) formed by projecting the detection target space R illustrated in FIG. 1 on the panel 31, and is larger than the detection target region R_{DEC} by a predetermined ratio. In the following description, the ratio of the arrangement region R_{POS} to the detection target region R_{DEC} is denoted by a ratio "S" (S ≥ 100%) of the length of one side. In the present application, when the center of a sensing coil 32 is located on the outer periphery of the arrangement region R_{POS}, the sensing coil 32 is regarded as being arranged in the arrangement region R_{POS}.

For example, as illustrated in FIG. 4, if the size of the detection target region R_{DEC} is set to 300 mm × 300 m and the length of one side of the arrangement region R_{POS} for the sensing coils 32 is aligned with the detection target region R_{DEC}, the ratio S is 100%. In FIG. 4, eight sensing coils 32 are arranged on the outer periphery of the arrangement region R_{POS} and one sensing coil 32 is arranged on the center of the detection target region R_{DEC}.

As illustrated in FIG. 5, if the size of the detection target region R_{DEC} is set to 300 mm × 300 mm and the size of the arrangement region R_{POS} for the sensing coils 32 is set to 420 mm × 420 m, the ratio S is 140%. In FIG. 5, eight sensing coils 32 are arranged only on the outer periphery of the arrangement region R_{POS}.

Preferably, at least some of the sensing coils 32 may be arranged in an intermediate region (third region) R_{MD} which is a region located between the outer periphery of the arrangement region R_{POS} and the outer periphery of the detection target region R_{DEC} (refer to FIGS. 4 and 5). In other words, the intermediate region R_{MD} is a region located on the inner peripheral side of the arrangement region R_{POS} and located on the outer peripheral side of the detection target region R_{DEC}. A sensing coil 32 may be arranged or may not be arranged within the detection target region R_{DEC}. However, preferably, at least one sensing coil 32 may be arranged within the detection target region R_{DEC} and at least one sensing coil 32 may be arranged within the intermediate region R_{MD} (refer to FIG. 4).

Preferably, the sensing coils 32 may be line-symmetric with respect to an axis that passes through the center of the arrangement region R_{POS} or rotationally-symmetric with respect to the center of the arrangement region R_{POS}.

The sensing coils 32 are arranged with their central axes 32a parallel to each other. In other words, openings of the sensing coils 32 face the same direction. More preferably, the sensing coils 32 may be arranged with the central axes 32a parallel to the vertical direction (Z direction), that is, with opening faces substantially parallel to the XY plane. In other words, the sensing coils 32 are arranged with magnetic field detection directions perpendicular to the principal face of the panel 31.

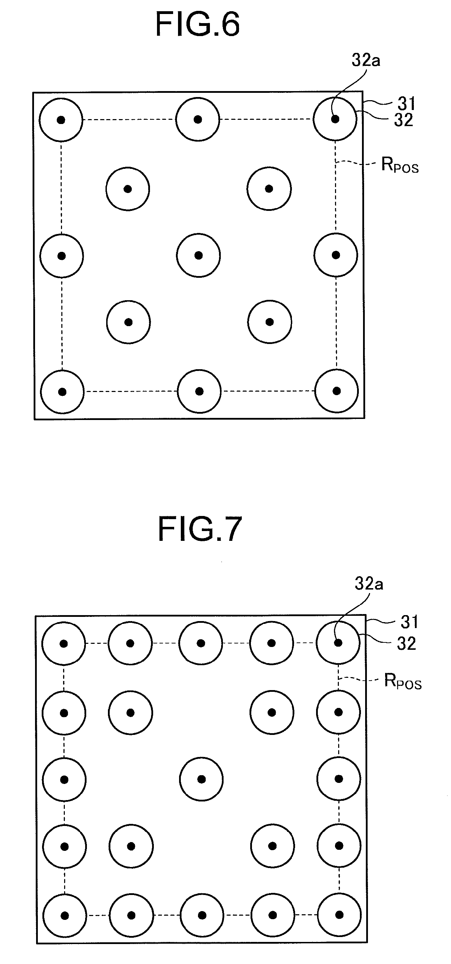

FIGS. 6 to 9 are schematic diagrams illustrating other examples of the arrangement of sensing coils 32 in the position detection device according to the first embodiment. FIG. 6 illustrates an example in which 13 sensing coils 32 are arranged with a uniform arrangement density in the entire arrangement region R_{POS}. In other words, the distance between one sensing coil 32 and another sensing coil 32 that is closest thereto (more strictly, the distance between the centers of the sensing coils 32, hereinafter referred to as an adjacent coil distance) is constant in the arrangement region R_{POS}.

FIG. 7 illustrates an example in which 21 sensing coils 32 are arranged with a higher arrangement density in the peripheral part than the central part of the arrangement region R_{POS}. In other words, the adjacent coil distance is shorter in the peripheral part than the central part of the arrangement region R_{POS}. Alternatively, the arrangement density may increase (the adjacent coil distance may decrease) from the central part toward the peripheral part of the arrangement region R_{POS}.

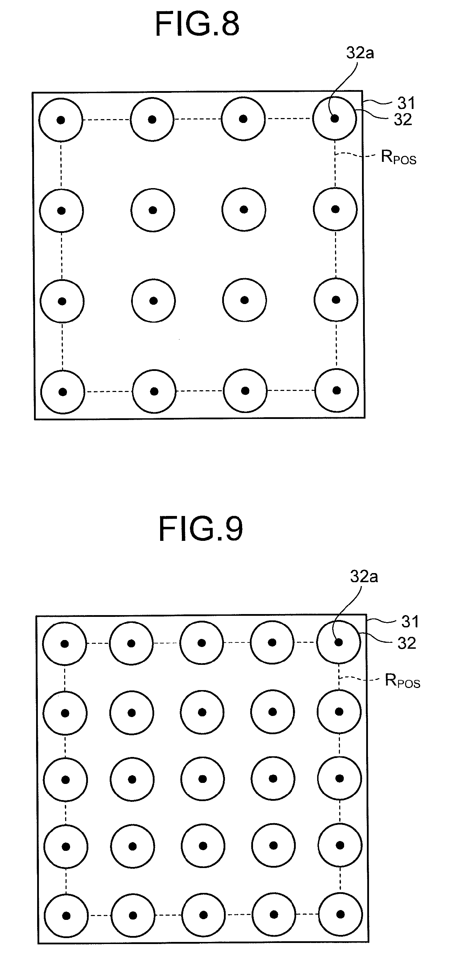

FIG. 8 illustrates an example in which 16 sensing coils 32 are arrange in matrix with a uniform arrangement density in the entire arrangement region R_{POS}. That is, in this example, the adjacent coil distance is constant. A sensing coil 32 is not necessarily arranged on the center of the arrangement region R_{POS}.

FIG. 9 illustrates an example in which 25 sensing coils 32 are arrange in matrix with a uniform arrangement density in the entire arrangement region R_{POS} and a higher arrangement density than the case of FIG. 8. That is, in this example, the adjacent coil distance is constant and shorter than the case of FIG. 8. The examples of FIGS. 8 and 9 differ from each other not only in the arrangement density but also in that no sensing coil 32 is arranged on the central position of the arrangement region R_{POS} in the example of FIG. 8 but a sensing coil 32 is arranged on the central position of the arrangement region R_{POS} in the example of FIG. 9.

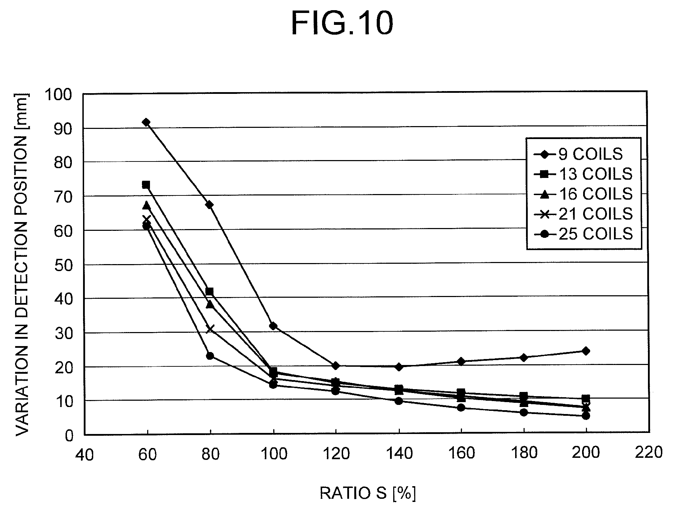

FIG. 10 is a graph illustrating a result of a simulation of a variation in three-dimensional detection position of the capsule endoscope 10 when the ratio S of the arrangement region R_{POS} to the detection target region R_{DEC} is changed in the arrangement of sensing coils 32 illustrated in each of FIGS. 4 to 9. As illustrated in FIG. 10, the detection position variation is sharply reduced when the ratio S is 100% or more. When the ratio S is 120% or more, the variation is effectively reduced regardless of the number of arranged sensing coils 32. Practically, the ratio S is preferably 120% to 200%, and more preferably 120% to 140% in which the detection position variation can be reduced in the most effective manner.

As described above, in the first embodiment of the present invention, a plurality of sensing coils 32 is arranged in the arrangement region R_{POS} having a size of 100% or more of the detection target region R_{DEC} for the capsule endoscope 10. Thus, even when the sensing coils 32 are arranged on the same plane, it is possible to accurately detect the three-dimensional position of the capsule endoscope 10. Therefore, it is possible to achieve space saving in the position detection device and the entire position detection system compared to a conventional structure.

### (FIRST MODIFICATION)

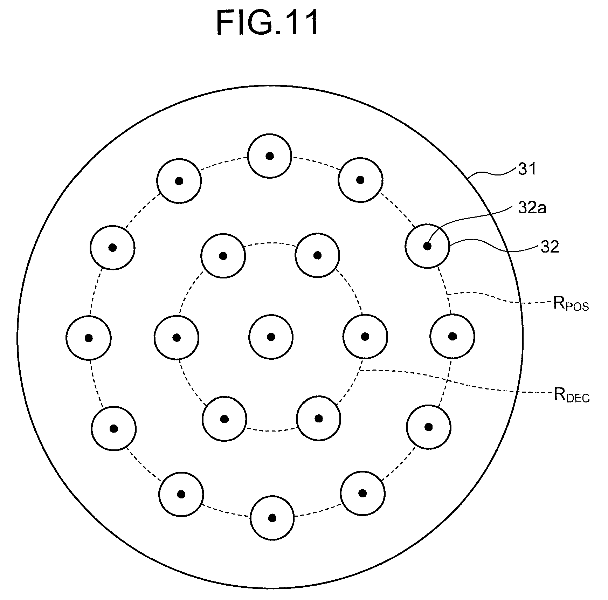

FIG. 11 is a schematic diagram illustrating the arrangement of sensing coils 32 in a first modification of the first embodiment. Although, in the first embodiment, the sensing coils 32 are arranged in a quadrangular shape, the shape of a region in which the sensing coils 32 are arranged is not particularly limited to any shape. For example, as illustrated in FIG. 11, the sensing coils 32 may be concentrically arranged. In this case, the size of the arrangement region R_{POS} may be defined to be 100% or more in radius or diameter with respect to the detection target region R_{DEC}.

### (SECOND MODIFICATION)

In the first embodiment, a guidance magnetic field for guiding the capsule endoscope 10 is generated by the external permanent magnet 41. Alternatively, a guidance magnetic field may be electromagnetically generated. In this case, the frequency of a guidance magnetic field may be set so as to prevent interference with a magnetic field for position detection generated by the capsule endoscope 10.

### (SECOND EMBODIMENT)

Next, a second embodiment of the present invention will be described.

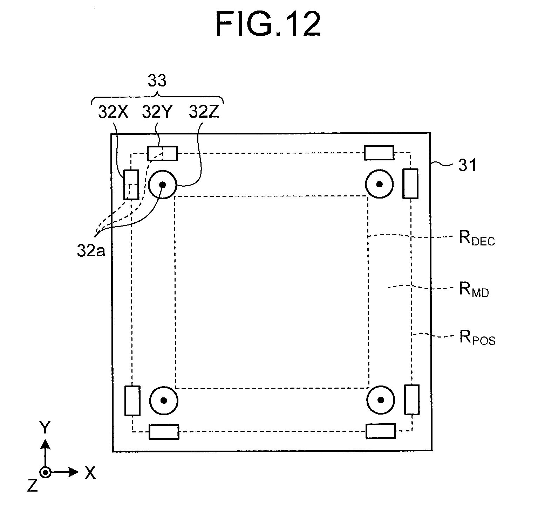

FIGS. 12 to 16 are schematic diagrams illustrating the arrangement of sensing coils 32 in a position detection device according to the second embodiment. The configuration and the operation of each unit of the position detection device and a position detection system according to the second embodiment are similar to those of the first embodiment excepting the arrangement of the sensing coils 32.

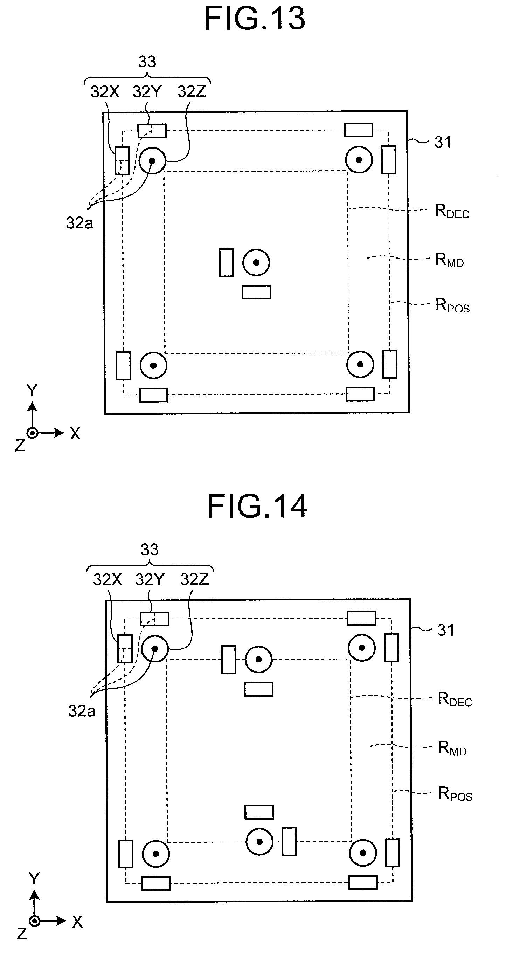

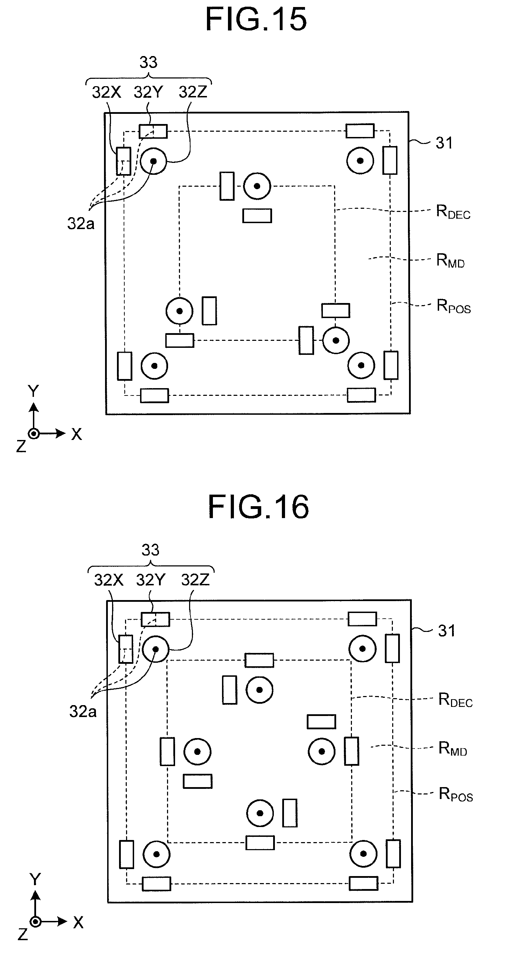

In the second embodiment, a set of three sensing coils 32 whose central axes 32a are parallel to the respective X, Y and Z axes of a panel 31 is defined as a coil set 33, and a plurality of coil sets 33 is arranged in an arrangement region R_{POS} whose length of one side is larger by a predetermined ratio than a detection target region R_{DEC}. FIG. 12 illustrates an arrangement example with four coil sets 33 (that is, 12 sensing coils 32). FIG. 13 illustrates an arrangement example with five coil sets 33 (that is, 15 sensing coils 32). FIG. 14 illustrates an arrangement example with six coil sets 33 (that is, 18 sensing coils 32). FIG. 15 illustrates an arrangement example with seven coil sets 33 (that is, 21 sensing coils 32). FIG. 16 illustrates an arrangement example with eight coil sets 33 (that is, 24 sensing coils 32).

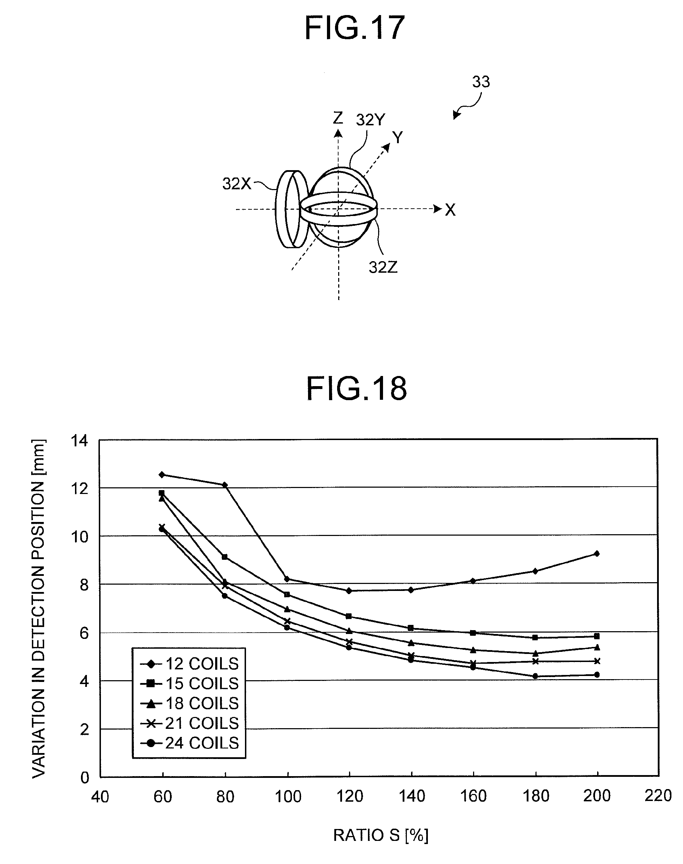

FIG. 17 is a perspective view of the coil set 33. As illustrated in FIG. 17, one coil set 33 includes a sensing coil 32X whose central axis is parallel to the X axis, a sensing coil 32Y whose central axis is parallel to the Y axis, and a sensing coil 32Z whose central axis is parallel to the Z axis. That is, one coil set 33 includes three sensing coils whose magnetic field detection directions are parallel to the respective X, Y, and Z axes. Such a configuration of the coil set 33 makes it possible to increase the arrangement density of sensing coils 32 (that is, reduce the adjacent coil distance) at the position of the coil set 33 and to three-dimensionally detect a change in a magnetic field at this position.

At least some of the coil sets 33 may be arranged in an intermediate region R_{MD} which is a region located between the outer periphery of the arrangement region R_{POS} and the outer periphery of the detection target region R_{DEC} (refer to FIG. 12) similarly to the first embodiment. A coil set 33 may be arranged or may not be arranged within the detection target region R_{DEC}. However, preferably, at least one coil set 33 may be arranged within the detection target region R_{DEC} and at least one coil set 33 may be arranged within the intermediate region R_{MD} (refer to FIG. 13) .

Preferably, the coil sets 33 may be line-symmetric with respect to an axis that passes through the center of the arrangement region R_{POS} or rotationally-symmetric with respect to the center of the arrangement region R_{POS}. Each of the coil sets 33 at each position may face any direction as long as the central axes 32a of the three sensing coils 32 are parallel to the respective X, Y and Z axes.

The arrangement density of the coil sets 33 may be uniform in the entire arrangement region R_{POS} or may be higher in the peripheral part than the central part of the arrangement region R_{POS}. In other words, the distance between one coil set 33 and another coil set 33 that is closest thereto (hereinafter referred to as an adjacent coil set distance) may be constant in the arrangement region R_{POS} or may be shorter in the peripheral part than the central part of the arrangement region R_{POS}. Preferably, the arrangement density of the coil sets 33 may increase (the adjacent coil set distance may decrease) from the central part toward the peripheral part of the arrangement region R_{POS}.

FIG. 18 is a graph illustrating a result of a simulation of a variation in three-dimensional detection position of the capsule endoscope 10 when the ratio S of the arrangement region R_{POS} to the detection target region R_{DEC} is changed in the arrangement of sensing coils 32 (coil sets 33) illustrated in each of FIGS. 12 to 16. As illustrated in FIG. 18, the detection position variation is sharply reduced when the ratio S is 100% or more. In particular, when the ratio S is 120% or more, the variation is effectively reduced regardless of the number of arranged sensing coils. Practically, when 12 sensing coils 32 are arranged (when no sensing coil 32 is arranged within the detection target region R_{DEC}: refer to FIG. 12), the ratio S is preferably 100% to 160%. When 15 to 24 sensing coils 32 are arranged (when a sensing coil 32 is arranged also within the detection target region R_{DEC}: refer to FIGS. 13 to 16), the ratio S is preferably 120% to 200%. Further, the ratio S is more preferably 120% to 140% in which the detection position variation can be reduced in the most effective manner. That is, when the ratio S is 120% to 140%, the detection position variation of the capsule endoscope 10 can be effectively reduced regardless of the number of arranged sensing coils 32.

As described above, in the second embodiment, a plurality of coil sets 33 capable of detecting a three-dimensional change in a magnetic field is arranged in the arrangement region R_{POS} having a size of 100% or more of the detection target region R_{DEC}. Thus, even when a plurality of sensing coils 32 is arranged on a single plane, it is possible to accurately detect the capsule endoscope 10 inside the subject 2.

Note that, in the second embodiment, a plurality of coil sets 33 may be arranged in a circular shape similarly to the first modification.

### (THIRD EMBODIMENT)

Next, a third embodiment of the present invention will be described.

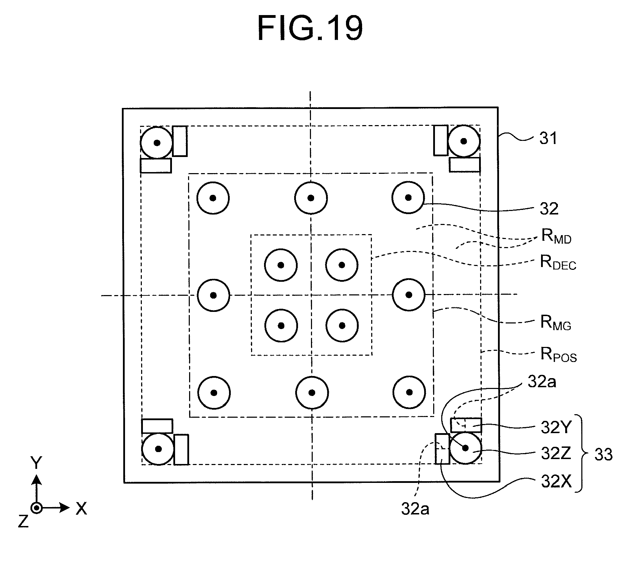

FIG. 19 is a schematic diagram illustrating an example of the arrangement of sensing coils 32 in a position detection device according to the third embodiment. The configuration and the operation of each unit of the position detection device and a position detection system according to the third embodiment are similar to those of the first embodiment excepting the arrangement of the sensing coils 32.

In the third embodiment, sensing coils 32 whose central axes 32a are parallel to the Z axis and coil sets 33 each of which includes three sensing coils 32 (sensing coils 32X, 32Y and 32Z) whose central axes 32a are parallel to the respective X, Y and Z axes are arranged in an arrangement region R_{POS} whose length of one side is larger by a predetermined ratio than a detection target region R_{DEC}.

Preferably, the sensing coils 32 may be arranged within the detection target region R_{DEC} with the central axes 32a parallel to the Z axis, and the coil sets 33 may be arranged in an intermediate region R_{MD} which is a region located between the outer periphery of the arrangement region R_{POS} and the outer periphery of the detection target region R_{DEC}. In particular, the coil sets 33 may be arranged at four corners of the arrangement region R_{POS}. This configuration makes it possible to increase the arrangement density of sensing coils 32 (reduce the adjacent coil distance) at the corners of the arrangement region R_{POS} and three-dimensionally detect a change in a magnetic field at this position.

When a coil set 33 that includes sensing coils 32 each having a large diameter is arranged above the movable range R_{MG} of the external permanent magnet 41 (refer to FIG. 3), the sensing coils 32 whose central axes are parallel to the respective X and Y axes in the coil set 33 may interfere with the operation of the external permanent magnet 41 by movement of the external permanent magnet 41 to the upper side in the Z direction. In this case, the distance between the external permanent magnet 41 and the detection target space R needs to be forced to be increased.

In view of the above, only sensing coils 32 whose central axes 32a are parallel to the Z axis are arranged in the detection target region R_{DEC} which is included in a region above the movable range R_{MG}. This configuration enables the interference between the sensing coils 32 and the external permanent magnet 41 to be prevented. Accordingly, it is possible to bring the permanent magnet 41 close to the detection target space R. Thus, even when the size of the external permanent magnet 41 is reduced, a magnetic field having a sufficient strength can be formed in the detection target space R. When there is no possibility of the sensing coils 32 interfering with the operation in the Z axis direction of the external permanent magnet 41, the coil set 33 may be arranged above the movable range R_{MC}.

In the third embodiment having such a configuration, the sensing coils 32 each of which is capable of one-dimensionally detecting a change in a magnetic field are arranged in the detection target region R_{DEC} which is a movable range of the capsule endoscope 10, and the coil sets 33 each of which is capable of three-dimensionally detecting a change in a magnetic field are arranged in the arrangement region R_{POS} away from the detection target region R_{DEC}. Thus, even when the plurality of sensing coils 32 is arranged on a single plane, it is possible to efficiently and accurately detect the detection of the capsule endoscope 10.

Coil sets 33 are not necessarily arranged at all of the four corners of the arrangement region R_{POS}. For example, a coil set 33 may be arranged at least at one corner, and sensing coils 32 whose central axes 32a are parallel to the Z axis may be arranged at the other corners. Also in this case, it is possible to improve the accuracy of detecting the capsule endoscope 10 introduced into the subject 2 compared to a conventional structure.

Also in the third embodiment, a plurality of sensing coils 32 and coil sets 33 may be arranged in a circular shape similarly to the first modification.

### (THIRD MODIFICATION)

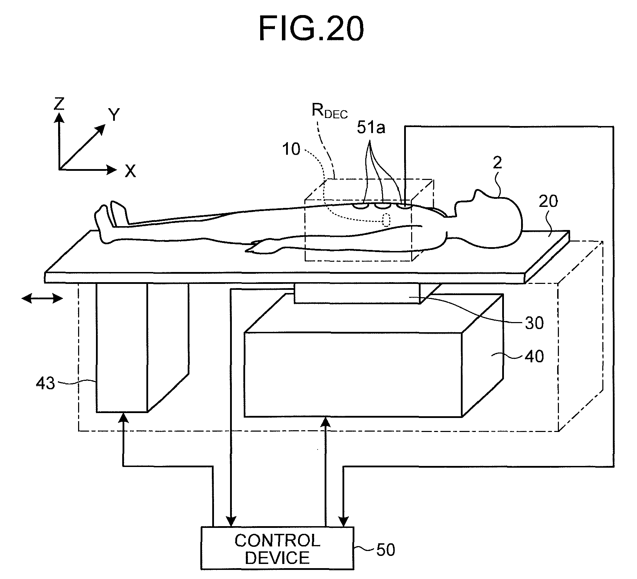

FIG. 20 is a schematic diagram illustrating a third modification of the position detection systems according to the first to third embodiments of the present invention. In the position detection system illustrated in FIG. 1, the bed 20 is fixed, and the external permanent magnet 41 is moved by the magnetic field driver 42 to change the relative position between the external permanent magnet 41 and the subject 2. However, the subject 2 may be moved with the position of the external permanent magnet 41 fixed, or both the subject 2 and the external permanent magnet 41 may be moved in opposite directions to achieve a change in the relative position therebetween.

For example, as illustrated in FIG. 20, a translation driver 43 which translates the bed 20 along the X direction may be provided to enable movement of the bed 20 under the control of the control unit 56. In this case, for example, in order to move the capsule endoscope 10 in a plus X direction with respect to the subject 2, the bed 20 on which the subject 2 is placed is moved in a minus X direction. Accordingly, the capsule endoscope 10 can be moved in the plus X direction relative to the subject 2.

In this case, it is preferable that the magnetic field detection device 30 is fixed under the bed 20 or inside the bed 20 and moved integrally with the bed 20.

### (FOURTH MODIFICATION)

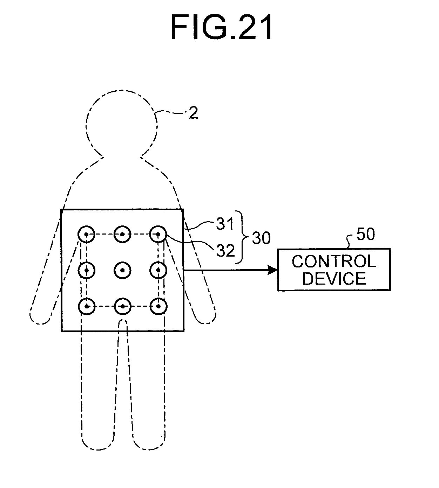

FIG. 21 is a schematic diagram illustrating a fourth modification of the position detection systems according to the first to third embodiments of the present invention. In the above first to third embodiments, the subject 2 is placed on the bed 20, and the magnetic field detection device 30 is placed under the bed 20 with the principal face of the panel 31 parallel to the horizontal plane. However, the placement of the magnetic field detection device 30 is not particularly limited to any position as long as an alternating magnetic field generated from the magnetic field generator 14 incorporated in the capsule endoscope 10 introduced into the subject 2 can be detected. For example, as illustrated in FIG. 21, when an examination is performed with the subject 2 standing upright or seated, the panel 31 may be placed near the subject 2 with the principal face parallel to the vertical direction.

The above first to third embodiments and first to fourth modifications are merely examples for carrying out the present invention, and the present invention is not limited to these embodiments and modifications. The present invention may create various inventions by appropriately combining a plurality of elements disclosed in the first to third embodiments and first to fourth modifications. The present invention can be modified in various manners in accordance with specifications. Further, it is obvious from the above description that other various embodiments are practicable within the scope of the present invention.

### Reference Signs List

- 1: position detection system
- 2: subject
- 10: capsule endoscope
- 11: imaging unit
- 12: control unit
- 13: transmission unit
- 14: magnetic field generator
- 15: power supply unit
- 16: permanent magnet
- 20: bed
- 30: magnetic field detection device
- 31: panel
- 32, 32X, 32Y, 32Z: sensing coil
- 32a: central axis
- 33: coil set
- 40: guidance magnetic field generation device
- 41: external permanent magnet
- 42: magnet driver
- 43: translation driver
- 50: control device
- 51: receiving unit
- 51a: receiving antenna
- 52: operation input unit
- 53: output unit
- 54: storage unit
- 55: signal processor
- 56: control unit
- 101: casing
- 102: cylindrical casing
- 103, 104: dome-shaped casing
- 111: illumination unit
- 112: optical system
- 113: imager
- 141: transmission coil
- 142: capacitor
- 421: plane position changing unit
- 422: vertical position changing unit
- 423: elevation angle changing unit
- 424: turning angle changing unit
- 551: filter unit
- 552: amplifier
- 553: A/D converter
- 561: image processor
- 562: positional information generation unit
- 562a: FFT processing unit
- 562b: position calculation unit
- 563: guidance magnetic field controller

## Claims

1. A position detection device for detecting a position of a capsule medical device, the capsule medical device having therein a magnetic field generator for generating a magnetic field, the position detection device comprising:
a plurality of magnetic field detection units each configured to detect the magnetic field generated by the magnetic field generator and to output a detection signal; and
a position calculation unit configured to calculate the position of the capsule medical device based on the detection signal output from each of the magnetic field detection units, wherein
the magnetic field detection units are arranged on a single plane, the plane having: a first region formed by projecting a closed detection target space for detecting the capsule medical device on the plane; and a second region that is a closed region on the plane and includes the first region and is larger than the first region by a predetermined ratio, wherein
the magnetic field detection units are arranged in the second region.

2. The position detection device according to claim 1, wherein at least one of the magnetic field detection units is arranged in a third region located between an outer periphery of the first region and an outer periphery of the second region.

3. The position detection device according to claim 2, wherein at least one of the magnetic field detection units is arranged in the first region and at least another one of the magnetic field detection units is arranged in the third region.

4. The position detection device according to claim 1, wherein at least some of the magnetic field detection units are arranged with directions for detecting the magnetic field parallel to an axis perpendicular to the plane.

5. The position detection device according to claim 1, wherein at least some of the magnetic field detection units are arranged in such a manner that three of the magnetic field detection units whose directions for detecting the magnetic field are respectively parallel to two axes perpendicular to each other within the plane and an axis perpendicular to the plane are defined as one set.

6. The position detection device according to claim 5,
wherein some of the magnetic field detection units are arranged in the first region with the directions for detecting the magnetic field parallel to the axis perpendicular to the plane, and
some other of the magnetic field detection units are arranged in a third region located between an outer periphery of the first region and an outer periphery of the second region in such a manner that three of the magnetic field detection units whose directions for detecting the magnetic field are respectively parallel to the two axes perpendicular to each other within the plane and the axis perpendicular to the plane are defined as one set.

7. The position detection device according to claim 1, wherein nearest neighbor distances between the magnetic field detection units are constant.

8. The position detection device according to claim 1, wherein a nearest neighbor distance between any two of the magnetic field detection units in a peripheral part of the second region is shorter than a nearest neighbor distance between any two of the magnetic field detection units in a central part of the second region.

9. The position detection device according to claim 1,
wherein the second region has a quadrangular shape, and
a nearest neighbor distance between any two of the magnetic field detection units at a corner of the quadrangular shape of the second region is shorter than a nearest neighbor distance between any two of the magnetic field detection units in a central part of the second region.

10. The position detection device according to claim 1, wherein the ratio is 120% or more.

11. The position detection device according to claim 10, wherein the ratio is 120% or more and 200% or less.

12. The position detection device according to claim 11, wherein the ratio is 120% or more and 140% or less.

13. A position detection system comprising:
the position detection device according to claim 1; and
a capsule medical device having therein a magnetic field generator configured to generate a magnetic field.

14. The position detection system according to claim 13, further comprising a bed for placing thereon a subject into which the capsule medical device is configured to be introduced,
wherein the magnetic field detection units are arranged under the bed integrally with the bed with the plane parallel to a surface of the bed on which the subject is configured to be placed.

15. The position detection system according to claim 13, wherein the capsule medical device further has therein a permanent magnet, and
the position detection system further comprises:
a guidance magnetic field generator that is disposed outside a subject into which the capsule medical device is configured to be introduced, the guidance magnetic field generator being configured to generate a magnetic field in a space including the first region; and
a guidance magnetic field controller configured to at least one of translate and rotate the guidance magnetic field generator to change a guidance magnetic field at a position of the capsule medical device, and thereby to guide the capsule medical device in the subject.

16. The position detection system according to claim 15, wherein the first region is smaller than a range in which the guidance magnetic field generator is movable within a horizontal plane.

17. The position detection system according to claim 13, wherein the magnetic field generator is configured to generate an alternating magnetic field having a predetermined frequency component.
